# EUROPÄISCHE PATENTSCHRIFT

(11) **EP 0 692 983 B1**
(45) Veröffentlichungstag und Bekanntmachung des Hinweises auf die Patenterteilung: **30.09.1998**
(21) Anmeldenummer: 94911099.3
(22) Anmeldetag: 31.03.1994
(51) Int. Cl.: A61L 2/26, G01N 31/22

(54) **LEBENSDAUERINDIKATOR FÜR WIEDERHOLT STERILISIERBARE KUNSTSTOFFPRODUKTE**
SHELF-LIFE INDICATOR FOR REPEATEDLY STERILIZABLE PLASTIC PRODUCTS
INDICATEUR DE DURABILITE POUR PRODUITS EN MATIERE PLASTIQUE RESTERILISABLES

(30) Priorität: 10.04.1993 DE 4311846
(43) Veröffentlichungstag der Anmeldung: 24.01.1996
(73) Patentinhaber: Haindl, Hans, Dr., D-30974 Wennigsen (DE)
(72) Erfinder: Haindl, Hans, Dr., D-30974 Wennigsen (DE)
(74) Vertreter: Körner, Peter
(86) Internationale Anmeldenummer: DE9400374
(87) Internationale Veröffentlichungsnummer: WO9423763

(56) Entgegenhaltungen:
- EP-A- 0 069 037
- EP-A- 0 581 400
- DE-A- 2 841 749
- US-A- 3 932 134
- US-A- 4 038 873
- US-A- 4 533 640
- US-A- 4 737 463

## Beschreibung

Die Erfindung betrifft einen Lebensdauerindikator für wiederholt sterilisierbare Kunststoffprodukte gemäß dem Anspruch 1.

Als Beitrag zur Abfallvermeidung werden in der Medizin zunehmend Produkte eingesetzt, die nur noch zum Teil Einwegprodukte sind und deren andere Teile wiederverwendet werden. Diese werden in der Regel aus dampfsterilisierbaren Kunststoffen hergestellt.

Ein Problem bei diesen Produkten ist, daß die Lebensdauer der dampfsterilisierbaren Kunststoffprodukte begrenzt ist. Dadurch besteht die Gefahr, daß diese nach wiederholter Sterilisation während des Gebrauchs versagen. Dies könnte unter Umständen nachteilige Folgen haben, dadurch, daß Bruchteile in Wundgebiete fallen oder daß ein wichtiger Arbeitsschritt des Operateurs wegen Versagen des Instruments nicht zu Ende geführt werden kann.

Derartige Produkte werden in der Regel so ausgelegt, daß sie ca. 100 Sterilisationen überstehen, ohne Schaden zu nehmen. Es ist naheliegend, nach einem Indikator zu suchen, der dem Anwender signalisiert, daß das Produkt aufgebraucht ist und nicht mehr weiterbenutzt werden sollte.

Diese Aufgabenstellung ist jedoch nicht einfach zu lösen. Die Produkte werden in der Regel vor dem Resterilisieren in automatischen Waschmaschinen mit teilweise sehr abrasiven Reinigungsmitteln behandelt. Ein auf der Oberfläche angebrachter Indikator würde in jedem Falle in seiner Reaktion mehr von den Reinigungsmitteln abhängen, als von der eigentlichen Sterilisation. Deshalb sind Versuche, Farbindikatoren auf der Oberfläche anzubringen, bisher auch erfolglos verlaufen.

Aus der DE 31 26 275 A1 ist es bekannt, medizinische Geräte mit Indikatoren für Sterilisationsverfahren auszustatten. Als Indikator dient ein Farbstoff, der unter dem Einfluß des angewandten Sterilisationsverfahrens seine Farbe ändert und so signalisiert, daß eine Sterilisation erfolgt ist. Die Indikatoren eignen sich nur für Einwegprodukte.

In der JP 4-364174 A, Patents Abstracts of Japan, Sec. C, Vol 17, No. 232 (C-1056) ist außerdem eine Indikatorzusammensetzung für Sterilisationsverfahren bei medizinischen Geräten beschrieben, die sowohl für Dampfsterilisation als auch für Sterilisation mit Äthylenoxid geeignet ist.

Ferner ist aus der DE 29 29 582 A1 eine sterilisierbare Verpackung für Gegenstände zur Anwendung im medizinischen Bereich bekannt. Die Verpackung besitzt im geschlossenen Inneren einen Feuchtigkeitsindikator in Form einer von außen sichtbaren Farbe, die sich bei Eindringen von Feuchtigkeit bleibend verändert.

Schließlich ist es aus der DE 31 03 936 A1 für sich bekannt, Polymermaterialien einzufärben, indem ein Farbstoff durch Diffusion aus einer Lösung oder aus der Gasphase in das transparente Polymermaterial überführt wird.

Der Erfindung liegt die Aufgabe zugrunde, einen Indikator anzugeben, der signalisiert, daß das sterilisierbare Produkt nicht mehr weiterbenutzt werden sollte.

Diese Aufgabe wird durch einen Lebensdauerindikator mit den Merkmalen im Anspruch 1 gelöst.

Die Wirkung der Erfindung, daß sich die in das Polymer eingebrachten Pigmente unter wiederholter Wärmebelastung verteilen und/oder verändern, beruht auf folgendem Verhalten. Die Pigmente diffundieren temperaturabhängig intermolekular in das Polymer. Die Wanderung der Moleküle erfolgt dabei dreidimensional, wodurch die ursprüngliche Konzentration am Ort der Einbringung abnimmt. Hinzukommt, daß im Zuge der wiederholten Wärmebelastung des Polymers reaktionsfreudige Zerfallsprodukte entstehen, die die Pigmente angreifen und dabei verändern. Unter Verändern wird hierbei jede Art der Veränderung bis zur Zerstörung verstanden. Auch hierdurch nimmt die Konzentration der Pigmente ab.

Die Anteile, die die Diffusion und die Zerstörung der Pigmente an ihrer Konzentration am ursprünglichen Einbringungsort haben, ist von der Art der Pigmente und des Polymers abhängig. Da beide Effekte in die gleiche Richtung gehen, ist die Kenntnis ihrer Anteile in der Praxis von untergeordneter Bedeutung. Jedenfalls bewirkt jede Dampfsterilisation ein geringfügiges Weiterdiffundieren der Pigmente und eine Veränderung einzelner Pigmente, so daß die Pigmentierung mit jeder Sterilisation zunächst unmerklich, später merklich blasser wird. Bei geeigneter Abstimmung zwischen Polymer und Pigment kann erreicht werden, daß die Pigmentierung nach ca. 100 Sterilisationen verschwunden ist.

Da die Pigmente in das Polymer eingebracht sind und beim Weiterdiffundieren immer tiefer in das Polymer hineindringen, können sie durch oberflächliche Reinigungsprozesse nicht entfernt oder beeinträchtigt werden.

Hinsichtlich der Anordnung der Pigmente im Polymer bestehen mehrere Möglichkeiten. So können die Pimente anfänglich gleichmäßig verteilt an oder nahe der sichtbaren Oberfläche eingebracht sein. Die Farbintensität nimmt dann im Laufe der Sterilisationen ab, da die Pigmente immer weiter in tiefere Schichten vordringen oder verändert werden, wodurch ihre Konzentration an der Oberfläche abnimmt. Ohne Vergleich ist es hier aber schwierig, die Farbintensität zu bestimmen, bei der das sterilisierbare Produkt nicht mehr weiterverwendet werden soll.

Weiterhin können die Pigmente anfänglich ungleichmäßig verteilt an oder nahe der sichtbaren Oberfläche eingebracht sein. Besonders zweckmäßig ist es hierbei, die Pigmente in Form einer Schrift und/oder geometrischen Figur einzubringen. Bei jeder Sterilisation geht die Konturenschärfe dieser Art Pigmentierung zurück, bis schließlich alle ursprünglich diskreten Flächen ineinandergeflossen sind. Diese Ausgestaltung ermöglicht eine wesentlich bessere Bestimmung des Zeitpunkts, ab dem das sterilisierbare Produkt nicht mehr weiterverwendet werden soll.

Als Beispiel hierfür könnte ein Schriftzug "reusable" oder "wiederverwendbar" eingebracht sein. Die Pigmente sind dabei an vorgegebenen Stellen mit hoher Konzentration und an den übrigen Stellen nicht vorhanden. So ergeben sich zuerst scharfe Konturen der Buchstaben, die mit zunehmender Diffusion immer unschärfer werden und schließlich miteinander verschmelzen, so daß der Schriftzug nicht mehr lesbar ist.

Zusätzlich kann das Polymer lichtstreuende und/oder lichtabsorbierende Eigenschaft aufweisen. Durch diese Maßnahme wird die Sichtbarkeit von in die Tiefe des Polymers vorgedrungener Pigmente verhindert, so daß die Abnabme der Farbintensität mit jeder Sterilisation beschleunigt wird.

Alternativ könnten die Pigmente entfernt von der sichtbaren Oberfläche eingebracht sein, wenn das Polymer optisch klar ist. Bei ursprünglich gleichmäßig verteilten Pigmenten wird eine wahrnehmbare Verblassung dann im wesentlichen durch Veränderung der Pigmente hervorgerufen, während bei ursprünglich ungleichmäßig verteilten Pigmenten die anfängliche Konturschärfe durch Diffusion der Pigmente zurückgeht.

Als Trägerstoffe für die Pigmente sind vorzugsweise Polymere aus der Gruppe der Polyamide, Polyester, Polyacetale, Polysulfone und Polyimide verwendbar. Diese Polymere sind bei den üblichen Sterilisationstemperaturen formbeständig und ermöglichen den Pigmenten eine Diffusionsgeschwindigkeit, bei der sich der Grad des Verblassens oder Verlaufens der Farben als Kriterium für das Ende der Benutzbarkeit der wiederverwendbaren Kunststoffprodukte auswerten läßt.

Eine Weiterbildung sieht vor, daß das Polymer mit Füllstoffen versehen ist. Ein als Füllstoff geeigneter Stoff ist Talkum (Siliziumoxid). Es hat sich gezeigt, daß dadurch das Diffusionsverhalten von Polymeren, die im ungefüllten Zustand nur eine geringe Diffusion der Pigmente zulassen, wesentlich verbessert werden kann und daß sich Füllstoffe im übrigen dazu eignen, das Verblassen der Pigmente bei jeder Sterilisation auf die zulässige Anzahl von Sterilisationszyklen abzustimmen.

Als Pigmente eignen sich insbesondere Azofarbstoffe, z. B. der Azofarbstoff C. I. Disperse Yellow 64. Azofarbstoffe sind bei den üblichen Sterilisationstemperaturen für sich temperaturbeständig und in der Lage, innerhalb der Polymere zu diffundieren. Polymere und Azofarbstoffe sind in ihren Eigenschaften also so aufeinander abgestimmt, daß durch ihre Kombination eine praxisgerechte Realisierung des Lebensdauerindikators ermöglicht wird.

Besonders vorteilhaft sind als Pigmente dienende Farbstoffe, die bei wiederholter Wärmebelastung von einer Farbe in eine andere Farbe umschlagen. Durch den Farbumschlag läßt sich besonders deutlich das Ende der Benutzbarkeit der wiederverwendbaren Kunststoffprodukte signalisieren. Auch dieser Effekt beruht auf dem Einfluß reaktionsfreudiger Zerfallsprodukte, die im Zuge der wiederholten Wärmebelastung des Polymers, insbesondere bei Polyacetalen entstehen, die die Pigmente angreifen und dabei verändern. Die Veränderung führt hier aber nicht zur Zerstörung der Pigmente, sondern zu einer Farbänderung oder einem Farbumschlag.

In einer besonders günstigen Ausführungsform kann man die Tatsache nutzen, daß das Pigment in verschiedenen Kunststoffen verschieden schnell diffundiert. So kann man z.B. durch einen zweischichtigen Aufbau eines schwer diffundierenden Kunststoffes an der Oberfläche und eines darunterliegenden leicht diffundierenden Kunststoffes den Abtransport des Pigmentes in die Tiefe beschleunigen. Hierdurch kommt es zu einem klaren Farbumschlag, da sich das Pigment nicht parallel zur Oberfläche verbreitet, sondern vorzugsweise in Tiefenrichtung.

Bei dem mehrschichtigen Aufbau können die Polymerschichten klebstofffrei, z.B. durch Ultraschallschweißen, Reibschweißen, Hochfrequenzschweißen, Thermokontaktschweißen miteinander verbunden sein. Dadurch wird ein unmittelbarer Übergang von einer zur anderen Schicht ohne störende Zwischenschicht erreicht.

Vorzugsweise werden die Pigmente nach Aufbringung auf die Oberfläche des Polymer per intermolekularer Diffusion in das Polymer eingebracht. Dadurch lassen sich die Pigmente zunächst oberflächlich mit einem üblichen Druckverfahren auf das Polymer aufdrucken und gelangen anschließend unter Temperaturbeaufschlagung durch Diffusion in tiefere Bereiche, so daß sie vor oberflächlichem Abtrag geschützt sind. Dabei kann bei hohen Temperaturen eine hohe Farbdichte im Polymer erreicht werden. Geeignete Druckverfahren sind Tampondruck, Siebdruck, Offsetdruck oder Tintenstrahldruck.

Alternativ können die Pigmente auch durch Zwei-Komponenten-Spritzguß in das Polymer eingebracht sein. In diesem Fall entfällt die Notwendigkeit, des Polymer per intermolekularer Diffusion in das Polymer einzubringen.

Hinsichtlich der Verbindung des Lebensdauerindikators mit dem wiederverwendbaren Produkt bestehen vorzugsweise folgende Möglichkeiten. Der Lebensdauerindikator kann konstruktiver Teil des Produktes sein, indem Teile des Produktes aus dem als Träger für die Pigmente dienenden Polymeren gefertigt sind. Alternativ kann der Indikator als Aufkleber gestaltet sein, der unlösbar und hitzebeständig auf ein Produkt klebbar ist.

Nachfolgend wird ein Ausführungsbeispiel der Erfindung anhand der Zeichnung erläutert. In der Zeichnung zeigen:
- Fig. 1: einen schematischen Querschnitt durch einen Lebensdauerindikator im Neuzustand und
- Fig. 2: einen schematischen Querschnitt durch einen Lebensdauerindikator im verbrauchten Zustand.

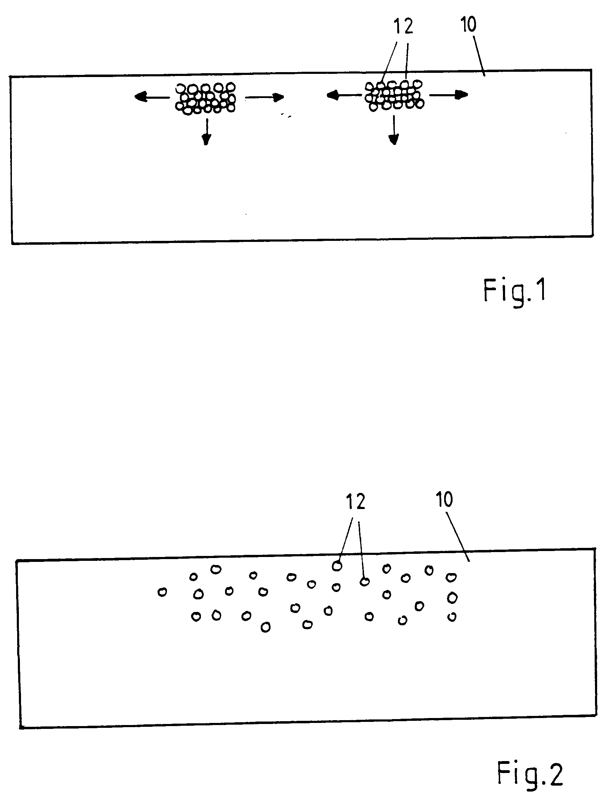

Die Figuren 1 und 2 zeigen einen Querschnitt durch einen Lebensdauerindikator. Dieser besteht aus dem als Träger dienenden Polymer 10 und Pigmenten 12. Von dem Polymer sind lediglich die äußeren Begrenzungen dargestellt, während die Pigmente als runde Körper gezeichnet sind. Die Darstellung gibt nicht die realen Größenverhältnisse der Pigmente zu dem Polymerkörper wieder, sondern ist rein schematisch.

Im Neuzustand gemäß Fig. 1 sind die Pigmente 12 in einem Kreisring in der Nähe der Oberfläche des Polymer 10 angeordnet. Bei jeder Sterilisation diffundieren die Pigmente 12 ein bißchen, was durch Pfeile angedeutet ist. Die Wanderung der Pigmente 12 erfolgt dabei in dreidimensionaler Richtung. Außerdem werden einige Pigmente verändert oder zerstört.

Nach einer Anzahl von Sterilisationsvorgängen, nach denen das Ende der Lebensdauer eines wiederverwendbaren Produktes erreicht ist, ergibt sich eine Verteilung der Pigmente 12 im Polymer 10, wie sie in Fig. 2 dargestellt ist. Die Pigmente sind nun verteilt und haben in ihrer Zahl abgenommen. Die ursprünglich scharf abgegrenzten Bereiche des Kreisringes, die mit Pigmenten 12 belegt waren sind verschwunden. Insbesondere die von verschiedenen Positionen in die Mitte des Kreisringes wandernden Pigmente 12 sind einander begegnet, so daß der ursprünglich freie Innenraum nun auch eingefärbt erscheint. Dies kann vom Benutzer als eindeutiges Kriterium gewertet werden, daß das Ende der Lebensdauer erreicht ist, das Produkt also nicht mehr verwendet werden sollte.

## Patentansprüche

1. Lebensdauerindikator, weicher mit einem wiederholt sterilisierbaren Kunststoffprodukt verbunden oder verbindbar ist, bestehend aus einem Polymer, in weiches Pigmente eingebracht sind, die durch Verteilung und/oder Veränderung bei wiederholter Wärmebelastung als Kriterium für das Ende der Lebensdauer auswertbar sind.

2. Lebensdauerindikator nach Anspruch 1, dadurch gekennzeichnet, daß die Pigmente anfänglich gleichmäßig verteilt an oder nahe der sichtbaren Oberfläche eingebracht sind.

3. Lebensdauerindikator nach Anspruch 1, dadurch gekennzeichnet, daß die Pigmente anfänglich ungleichmäßig verteilt an oder nahe der sichtbaren Oberfläche eingebracht sind.

4. Lebensdauerindikator nach Anspruch 3, dadurch gekennzeichnet, daß die Pigmente anfänglich in Form einer Schrift und/oder geometrischen Figur an oder nahe der sichtbaren Oberfläche eingebracht sind.

5. Lebensdauerindikator nach einem der Ansprüche 1 bis 4, dadurch gekennzeichnet, daß das Polymer lichtstreuende und/oder lichtabsorbierende Eigenschaft aufweist.

6. Lebensdauerindikator nach Anspruch 1, dadurch gekennzeichnet, daß die Pigmente entfernt von der sichtbaren Oberfläche eingebracht sind und daß das Polymer optisch klar ist.

7. Lebensdauerindikator nach einem der Ansprüche 1 bis 6, dadurch gekennzeichnet, daß das Polymer aus der Gruppe der Polyamide, Polyester, Polyacetale, Polysulfone und Polyimide besteht.

8. Lebensdauerindikator nach einem der Ansprüche 1 bis 7, dadurch gekennzeichnet, daß das Polymer mit Füllstoffen versehen ist.

9. Lebensdauerindikator nach einem der Ansprüche 1 bis 8, dadurch gekennzeichnet, daß als Pigmente Azofarbstoffe dienen.

10. Lebensdauerindikator nach einem der Ansprüche 1 bis 9, dadurch gekennzeichnet, daß als Pigmente Farbstoffe dienen, die bei wiederholter Wärmebelastung von einer Farbe in eine andere Farbe umschlagen.

11. Lebensdauerindikator nach einem der Ansprüche 1 bis 10, dadurch gekennzeichnet, daß das Polymer zweischichtig ist, wobei die eine Schicht schnellere Farbstoffdiffusionseigenschaften als die andere besitzt.

12. Lebensdauerindikator nach Anspruch 11, dadurch gekennzeichnet, daß die beiden Polymerschichten klebstofffrei miteinander verbunden sind, z.B. durch Ultraschallschweißen, Reibschweißen, Hochfrequenzschweißen, Thermokontaktschweißen.

13. Lebensdauerindikator nach einem der Ansprüche 1 bis 12, dadurch gekennzeichnet, daß die Pigmente nach Aufbringung auf die Oberfläche des Polymer per intermolekularer Diffusion in das Polymer eingebracht sind.

14. Lebensdauerindikator nach Anspruch 13, dadurch gekennzeichnet, daß die Pigmente durch Tampondruck, Siebdruck, Offsetdruck oder Tintenstrahldruck auf die Oberfläche des Polymer aufgebracht wurden.

15. Lebensdauerindikator nach einem der Ansprüche 1 bis 12, dadurch gekennzeichnet, daß die Pigmente durch ZweiKomponenten-Spritzguß in das Polymer eingebracht sind.

16. Lebensdauerindikator nach einem der Ansprüche 1 bis 15, dadurch gekennzeichnet, daß dieser konstruktiver Teil eines Produktes ist, indem Teile des Produktes aus dem als Träger für die Pigmente dienenden Polymeren gefertigt sind.

17. Lebensdauerindikator nach einem der Ansprüche 1 bis 15, dadurch gekennzeichnet, daß der Indikator als Aufkleber gestaltet ist, der unlösbar und hitzebeständig auf ein Produkt klebbar ist.

18. Verfahren zur Anzeige des Endes der Lebensdauer eines wiederholt sterilisierbaren Kunststoffproduktes mittels eines Indikators aus einem Polymer, in welches Pigmente eingebracht sind, die sich bei wiederholter Wärmebelastung verteilen und/oder verändern und dadurch als Kriterium für das Ende der Lebensdauer ausgewertet werden.

## Claims

1. Life indicator connected or connectable to a repeatedly sterilizable plastics product, comprising a polymer, into which are introduced pigments, which by distribution and/or modification, in the case of repeated thermal stressing, can be evaluated as a criterion for the end of life.

2. Life indicator according to claim 1, characterized in that the pigments are initially introduced in uniformly distributed manner on or close to the visible surface.

3. Life indicator according to claim 1, characterized in that the pigments are initially introduced in non-uniformly distributed manner on or close to the visible surface.

4. Life indicator according to claim 3, characterized in that the pigments are initially introduced in the form of a written character and/or geometrical figure on or close to the visible surface.

5. Life indicator according to one of the claims 1 to 4, characterized in that the polymer has light scattering and/or light absorbing properties.

6. Life indicator according to claim 1, characterized in that the pigments are introduced remote from the visible surface and that the polymer is optically clear.

7. Life indicator according to one of the claims 1 to 6, characterized in that the polymer is from the group of polyamides, polyesters, polyacetals, polysulphones and polyimides.

8. Life indicator according to one of the claims 1 to 7, characterized in that the polymer is provided with fillers.

9. Life indicator according to one of the claims 1 to 8, characterized in that azo dyes are used as pigments.

10. Life indicator according to one of the claims 1 to 9, characterized in that dyes are used as pigments and change from one colour to another in the case of repeated thermal stressing.

11. Life indicator according to one of the claims 1 to 10, characterized in that the polymer is in two-layer form, one layer having faster dye diffusion properties than the other.

12. Life indicator according to claim 11, characterized in that the two polymer layers are interconnected in adhesive-free manner, e.g. by ultrasonic, friction, high frequency or thermal contact welding.

13. Life indicator according to one of the claims 1 to 12, characterized in that following application to the surface of the polymer, the pigments are introduced into said polymer by intermolecular diffusion.

14. Life indicator according to claim 13, characterized in that the pigments are applied to the surface of the polymer by pad, screen, offset or ink jet printing.

15. Life indicator according to one of the claims 1 to 12, characterized in that the pigments are introduced into the polymer by two-component injection moulding.

16. Life indicator according to one of the claims 1 to 15, characterized in that it is a constructional part of a product, in that parts of the product are made from the polymer serving as a support for the pigments.

17. Life indicator according to one of the claims 1 to 15, characterized in that the indicator is in the form of a stick-on label, which can be stuck in non-detachable, heat-resistant manner to the product.

18. Method for indicating the end of the life of a repeatedly sterilizable plastics product by means of an indicator formed from a polymer, into which are introduced pigments, which are distributed and/or modified in the case of repeated thermal stressing and can consequently be evaluated as a criterion for the end of life.

## Revendications

1. Indicateur de durée de vie, qui est ou peut être lié à un produit en matière plastique restérilisable, consistant en un polymère dans lequel sont introduits des pigments qui peuvent être, pour la charge thermique repétée, exploités par l'intermédiaire de dispersion et/ou d'altération comme critère de la fin de la durée de vie.

2. Indicateur de durée de vie suivant la revendication 1, caractérisé en ce qu'initialement les pigments sont introduits, de manière uniforme, sur ou près de la surface visible.

3. Indicateur de durée de vie suivant la revendication 1, caractérisé en ce qu'initialement les pigments sont introduits dispersés, de manière non uniforme, sur ou près de la surface visible.

4. Indicateur de durée de vie suivant la revendication 3, caractérisé en ce qu'initialement les pigments sont introduits, sous forme d'un caractère et/ou de figure géométrique, sur ou près de la surface visible.

5. Indicateur de durée de vie suivant une des revendications 1 à 4, caractérisé en ce que le polymère présente une propriété de dif fusion et/ou d'absorption de la lumière.

6. Indicateur de durée de vie suivant la revendication 1, caractérisé en ce que les pigments sont introduits loin de la surface visible, et en ce que le polymère est optiquement clair.

7. Indicateur de durée de vie suivant une des revendications 1 à 6, caractérisé en ce que le polymère est constitué du groupe des polyamides, polyesters, polyacétates, polysulfones et polyimides.

8. Indicateur de durée de vie suivant une des revendications 1 à 7, caractérisé en ce que le polymère est pourvu de matières de charge.

9. Indicateur de durée de vie suivant une des revendications 1 à 8, caractérisé en ce que des colorants azoïques servent de pigments.

10. Indicateur de durée de vie suivant une des revendications 1 à 9, caractérisé en ce que, comme pigments, on se sert de colorants qui passent, lors de charge thermique répétée, d'une première couleur à une seconde couleur.

11. Indicateur de durée de vie suivant une des revendications 1 à 10, caractérisé en ce que le polymère est constitué par deux couches, la première couche ayant des propriétés de diffusion de colorant plus rapide que la seconde.

12. Indicateur de durée de vie suivant la revendication 11, caractérisé en ce que les deux couches de polymère sont liées l'une à l'autre, par exemple par soudure ultrasonique, soudure par friction, soudure haute fréquence, soudure par thermocontact.

13. Indicateur de durée de vie suivant une des revendications 1 à 12, caractérisé en ce que les pigments sont introduits, après épandage sur la surface du polymère, par diffusion intermoléculaire dans le polymère.

14. Indicateur de durée de vie suivant la revendication 13, caractérisé en ce que les pigments sont épandus sur la surface du polymère par tamponnage, sérigraphie, impression offset ou impression de jet d'encre.

15. Indicateur de durée de vie suivant une des revendications 1 à 12, caractérisé en ce que les pigments sont introduits dans le polymère par moulage par injection à deux composants.

16. Indicateur de durée de vie suivant une des revendications 1 à 15, caractérisé en ce qu'il est partie constructive d'un produit, étant donné que les parties du produits sont préparées à partir de polymères servant de support aux pigments.

17. Indicateur de durée de vie suivant une des revendications 1 à 15, caractérisé en ce que l'indicateur est une étiquette adhésive qui peut être collée, insoluble et résistante à la chaleur sur un produit.

18. Procédé pour indiquer la fin de la durée de vie d'un produit en matière plastique restérilisable au moyen d'un indicateur en polymère dans lequel des pigments sont introduits qui se dispersent et/ou se modifient lors de charges thermiques répétées et, ainsi, sont exploités comme critère pour la fin de la durée de vie.
